# EUROPÄISCHE PATENTANMELDUNG

(11) **EP 1 672 369 A1**
(43) Veröffentlichungstag der Anmeldung: **21.06.2006**
(21) Anmeldenummer: 04106641.6
(22) Anmeldetag: 16.12.2004
(51) Int. Cl.: G01N 33/86

(54) **Verfahren zur Bestimmung von Blutkoagulationsparametern, insbesondere in Durchflussmesssystemen**

(71) Anmelder: Roche Diagnostics GmbH, 68305 Mannheim (DE); F. HOFFMANN-LA ROCHE AG, 4002 Basel (CH)
(72) Erfinder: Schilling, Julia, Dr., 68167 Mannheim (DE); Ritter, Christoph, Dr., 8045 Graz (AT); Füllemann, Rainer, Dr., 69469 Weinheim (DE)
(74) Vertreter: Silber, Anton

(57) **Zusammenfassung**

Die Erfindung betrifft ein Verfahren und zur Bestimmung eines Blutkoagulations- oder Gerinnungsparameters aus einer flüssigen Probe, wobei die Probe mit einem ersten Reagenz (bzw. Reagenzbestandteil) in Kontakt gebracht wird, welches die Blutgerinnung initiiert und zu einem direkten oder indirekten, nachweisbaren Produkt führt, welches einen Rückschluss auf den Blutgerinnungstatus erlaubt, und der Probe oder dem Probe-Reagenz-Gemisch ein zweites Reagenz (bzw. Reagenzbestandteil) zugesetzt wird, welches die Bildung eines Blutgerinnsels bzw. eine Viskositätszunahme der Probe verlangsamt oder verhindert sowie ein entsprechendes Reagenz. Das erfindungsgemäße Verfahren und das dazugehörige Reagenz eignen sich insbesondere für Durchflussmesssysteme.

## Beschreibung

Die Erfindung betrifft ein Verfahren zur Bestimmung eines Blutkoagulations- oder Gerinnungsparameters aus einer flüssigen Probe, wobei die Probe mit einem ersten Reagenz (bzw. Reagenzbestandteil) in Kontakt gebracht wird, welches die Blutgerinnung initiiert und zu einem direkten oder indirekten, nachweisbaren Produkt führt, welches einen Rückschluss auf den Blutgerinnungstatus erlaubt, und der Probe oder dem Probe-Reagenz-Gemisch ein zweites Reagenz (bzw. Reagenzbestandteil) zugesetzt wird, welches die Bildung eines Blutgerinnsels bzw. eine Viskositätszunahme der Probe verlangsamt oder verhindert sowie ein entsprechendes Reagenz. Das erfindungsgemäße Verfahren und das dazugehörige Reagenz eignen sich insbesondere für Durchflussmesssysteme.

Gerinnungsphysiologische Untersuchungen finden in Krankenhäusern, Arztpraxen, Laboratorien u. a. zur prä-operativen Untersuchung, zur Erkennung von Blutungs- und Thromboserisiken, zur Überwachung der Antikoagulantien-Therapie bei thrombosegefährdeten Patienten und bei der Verlaufskontrolle zahlreicher Erkrankungen, z. B. schweren Infektionen, Leberfunktionsstörungen und malignen Erkrankungen, statt. Vermehrt wird die Bestimmung der Prothrombinzeit zum Selfmonitoring der Antikoagulantien-Therapie vom Patienten selbst durchgeführt.

Die am häufigsten durchgeführten Gerinnungstests sind Globaltests, bei denen gleichzeitig die Funktion mehrerer der am Gerinnungsvorgang beteiligten Faktoren und deren Zusammenwirken in der Gerinnungskaskade bestimmt werden. Als wichtigste Globalteste gelten der sog. Prothrombinzeit Test (abgekürzt PT), auch Quick Test genannt, der sog. aktivierte partielle Thromboplastinzeit Test (aPTT), sowie die Bestimmung der aktivierten Clotting - Time (ACT). Bei all diesen Tests wird die Zeit gemessen, die vom Start des entsprechenden Gerinnungsvorganges mit dem jeweiligen Reagenz bis zum Abschluss des Gerinnungsvorgangs vergeht. Der Gerinnungsvorgang gilt als abgeschlossen, wenn sich ein Gerinnsel gebildet hat. Typischerweise wird der Gerinnungsvorgang dabei über sekundäre, in der Probe beobachtbare Phänomene verfolgt (z. B. über Trübungsmessungen, Messung der Lichtstreuung, der Leitfähigkeit, der Viskosität oder der Aktivität von Schlüsselenzymen der Gerinnungskaskade, wie z. B. Thrombin).

Zur Bestimmung des Gerinnungsstatus von Blut- oder Plasmaproben ist dem Fachmann eine Vielzahl von Verfahren bekannt. Diese beruhen in der Regel darauf, dass einer entsprechenden Probe ein Aktivator des intrinsischen oder extrinsischen Gerinnungsweges zugesetzt, dadurch die eigentliche Gerinnungsreaktion gestartet und die dadurch einsetzende Veränderung der Probe (d.h. deren Gerinnung) beobachtet wird. Als Maß für den Gerinnungsstatus wird üblicherweise die Zeit herangezogen, die die Probe bis zum Gerinnen benötigt. Typischerweise wird das Gerinnen der Probe direkt oder indirekt beobachtet, beispielsweise durch Messung der mit der Gerinnung einhergehenden Viskositätsänderung der Probe (v. a. Viskositätsmessung, Messung von Ionen-, Partikel- oder Stoffbeweglichkeiten), durch Messung der mit der Gerinnung einhergehenden optischen Eigenschaften der Probe (v. a. Trübungsmessungen, Transmissionsmessungen), oder durch Bestimmung einzelner Enzymaktivitäten (z. B. photometrische oder elektrochemische Bestimmung der Aktivität des Thrombins, einer am Schluss der Gerinnungskaskade gebildeten Protease, durch Umsetzung entsprechender Proteasesubstrate (mit einem Peptidteil, der von Thrombin erkannt wird, und einer detektierbaren Gruppe) und Nachweis eines oder mehrerer Umsetzungsprodukte).

Der Fachmann unterscheidet je nach Fragestellung zwischen so genannten Globaltests, bei denen das Funktionieren des gesamten Gerinnungssystems geprüft wird, und Faktorentests, bei denen nur auf das Vorhandensein bzw. die Aktivität einzelner, an der Gerinnungskaskade beteiligter Faktoren (meist Enzyme) abgestellt wird.

Neben diesen Globaltests, bei denen die Gerinnungszeit als solche bestimmt wird und die zu diesem Zweck wie oben erwähnt beispielsweise die Aktivität der Protease Thrombin bestimmen, können mit entsprechenden, dem Fachmann bekannten alternativen Substraten andere Gerinnungstests, z. B. ein anti-Faktor Xa-Test, oder die Bestimmung von einzelnen Gerinnungsfaktoren realisiert werden. Hierfür ist es notwendig, die Reagenzien und die Testführung den jeweiligen "Zielanalyten" anzupassen. Beispielsweise kann es erforderlich sein, den Peptidteil des Substrats an die zu bestimmende Protease anzupassen.

Nachteilig an den Verfahren und Messsystemen des Standes der Technik ist, dass die gezielt gestartete Gerinnungsreaktion bis zur eigentlichen Gerinnung (d. h. bis zur Gerinnselbildung) abläuft. Die zu Beginn der Gerinnungsmessung flüssige Probe wird dabei in ein hochviskoses Material am Ende der Bestimmung verwandelt. Insbesondere für Durchflussmesssysteme, wie sie in der Laboranalytik häufig anzutreffen sind, sind die bekannten Verfahren deshalb nur sehr bedingt geeignet, da hier u. a. eine Gefahr der Verstopfung der Flüssigkeitskanäle besteht.

Aufgabe der vorliegenden Erfindung ist es, diese Nachteile zu beseitigen. Insbesondere ist es die Aufgabe der vorliegenden Erfindung, ein Verfahren und ein Reagenz bereitzustellen, mit dessen Hilfe in einem Durchflussmesssystem wiederholt Gerinnungsmessungen an Blutproben (oder an von Blut abgeleiteten Materialien) durchgeführt werden können.

Diese Aufgabe wird durch den Gegenstand der Erfindung gelöst.

Gegenstand der Erfindung ist ein Verfahren gemäß Anspruch 1, ein Reagenz gemäß Anspruch 11, sowie ein Koagulationsmesssystem gemäß Anspruch 17. Bevorzugte Ausführungsformen der Erfindung sind Gegenstand der abhängigen Ansprüche.

Das erfindungsgemäße Verfahren zur Bestimmung eines Gerinnungsparameters aus einer flüssigen Probe umfasst das In-Kontakt-Bringen der Probe mit einem ersten Reagenz. Dieses Reagenz setzt die Blutgerinnungskaskade in der Probe in Gang und initiiert so die Blutgerinnung. Um einen Rückschluss auf den Blutgerinnungstatus der Probe zu erlauben führen die Reagenzbestandteile, mit denen die Probenflüssigkeit reagiert, zu einem direkt oder indirekt nachweisbaren Produkt. Die Erfindung zeichnet sich dadurch aus, dass der Probe oder dem Probe-Reagenz-Gemisch mindestens ein zweites Reagenz zugesetzt wird, welches die Bildung eines Blutgerinnsels bzw. eine Viskositätszunahme der Probe verlangsamt oder verhindert. So wird es möglich, die Probe bzw. das Proben-Reagenz-Gemisch nach erfolgter Messung aus dem entsprechenden Reaktionsgefäß, insbesondere aus einem Durchflussmessgefäß, z. B. einem Probenkanal einer Durchflussmesszelle, durch Spülen zu entfernen. Bei Verfahren des Standes der Technik ist dies nicht ohne weiteres möglich, da die Gerinnung der Blutprobe auf die Reagenzzugabe erfolgt und das so erzeugte Gerinnsel durch einfaches Spülen in der Regel nicht zu beseitigen ist.

Wie einleitend dargestellt wird bei der Blutgerinnung am Ende der so genannten Gerinnungskaskade das Enzym Thrombin (eine Peptidase, die bestimmte Peptidbindungen spaltet) gebildet. Der Zusatz eines Thrombinsubstrats zum Reagenz erlaubt eine Thrombinaktivitätsbestimmung und damit Rückschluss auf den Status des Blutgerinnungssystems der Probe. Geeignete Thrombinsubstrate, die durch Thrombin gespalten werden und dabei detektierbare Spezies freisetzen, bestehen meist aus einem für Thrombin spezifischen Peptidteil, an den eine nachzuweisende, durch Thrombin abspaltbare Spezies gekoppelt ist. Solche Substrate sind dem Fachmann bekannt, beispielsweise aus US 6,352,630, US 5,108,890, US 4,797,472, US 5,190,862, EP-B 0 170 797, EP-B 0 076 042, US 2003/0146113 A1.

Ebenso bekannt sind Substanzen, die die Blutgerinnung starten, d. h. die Gerinnungskaskade, die aus mehreren voneinander teilweise abhängigen Reaktionsschritten besteht, initiieren. Je nachdem, ob der extrinsische oder der intrinsische Weg der Blutgerinnung angestoßen wird, spricht man bei den Substanzen von Aktivatoren des extrinsischen oder intrinsischen Wegs. Beispiele für solche Aktivatoren sind Thromboplastin, Kaolin, Celite, Sulfatide, Silicate, Ellagsäure etc.

Als Gerinnungsparameter, die mit dem erfindungsgemäßen Verfahren bestimmt werden können, kommen sowohl Globaltests (d. h. Tests, bei denen das Funktionieren des gesamten Gerinnungssystems geprüft wird, ohne auf einzelne Faktoren gezielt zu achten) als auch Tests für bestimmte Gerinnungsfaktoren in Frage. Bevorzugte Globaltests sind die Bestimmung der Prothrombinzeit (PT), der aktivierten Clottingzeit (ACT), der aktivierten partiellen Thromboplastinzeit (aPTT) und des TGT (Thrombin Generation Test). Bevorzugte Faktorentests sind Tests für die Faktoren Prothrombin, Faktor X, Faktor VIII, Faktor VII, Faktor IX, Protein C und deren aktivierte Formen etc. Die Bestimmung der Gerinnungsparameter erlaubt Rückschlüsse auf den Blutgerinnungstatus, d.h. eine Aussage darüber, ob das Blutgerinnungssystem desjenigen, von dem die Probe stammte, in Ordnung ist oder nicht, und gegebenenfalls auch darüber, welche Art von Störung vorliegt. Gerinnungstests werden auch eingesetzt zur Überprüfung von Medikamentenwirkungen, z. B. die Ecarin Clotting Time (ECT) und die Thrombin-Zeit (TT) zur Überprüfung der Wirkung direkter Thrombin-Inhibitoren.

Als Probe für das erfindungsgemäße Verfahren kommen alle üblicherweise in der Gerinnungsmessung verwendeten Probenmaterialien in Frage, insbesondere antikoaguliertes und nicht-antikoaguliertes Vollblut oder eine aus Vollblut abgeleitete oder daraus gewonnene Blutfraktion, insbesondere Plasma.

Erfindungsgemäß werden bei dem Verfahren zumindest zwei Reagenzien eingesetzt. Beide Reagenzien können dabei anfänglich bereits miteinander gemischt oder aber getrennt voneinander vorliegen. Im letzteren Fall können die mindestens zwei Reagenzien in beliebiger Reihenfolge mit der Probe in Kontakt gebracht werden. Wesentlich für den Rückschluss auf den Blutgerinnungsstatus ist, dass Reagenz und Probe zu einem nachweisbaren Produkt führen. Der Nachweis kann dabei mittels optischer Methoden (z. B. Absorptions-, Remissions-, Fluoreszenz-, Lumineszenz-, Trübungsmessung etc.) oder elektrochemisch (z. B. amperometrisch, coulometrisch, konduktometrisch, etc.) erfolgen. Reagenz und Probe können direkt zu einem messbaren Produkt(z. B. einem direkt detektierbaren Farbstoff oder einer direkt elektrochemisch nachzuweisenden Substanz, vgl. bspw. US 6,352,630, US 5,108,890, US 4,797,472, US 5,190,862, EP-B 0 170 797, US 2003/0146113 A1) führen. Es ist auch möglich, dass Probe und Reagenz zu einem direkten Produkt führen, welches zum Nachweis weiter umgesetzt werden muss (z. B. im Falle der enzymatischen Abspaltung einer Farbstoffvorstufe, die anschließend mit weiteren Reagenzbestandteilen zu einem Farbstoff weiterreagiert, welcher dann nachgewiesen werden kann, vgl. bspw. US 5,059,525, EP-B 0 076 042).

Vorzugsweise enthält das erste oder das zweite Reagenz ein Thrombinsubstrat, das zu einem direkt oder indirekt nachweisbaren Produkt führt (s. o.).

Das erste Reagenz bzw. der erste Reagenzbestandteil für das erfindungsgemäße Verfahren enthält einen Initiator oder Aktivator des intrinsischen oder extrinsischen Blutgerinnungsweges. Initiatoren bzw. Aktivatoren sind dem Fachmann bekannt. Beispielhaft seien die folgenden, erfindungsgemäß einsetzbaren Initiatoren bzw. Aktivatoren genannt: Thromboplastin (z. B. humaner, rekombinanter Tissue Faktor), Kaolin, Celite, Sulfatide, Silicate, Ellagsäure, etc. Dabei ist es für die Erfindung unerheblich, ob die Initiatoren bzw. Aktivatoren aus natürlichen Quellen oder synthetisch bzw. gentechnisch gewonnen werden.

Das zweite Reagenz bzw. der zweite Reagenzbestandteil bewirkt, dass die Bildung eines Blutgerinnsels bzw. eine Viskositätszunahme der Probe verlangsamt oder verhindert wird. Vorzugsweise wird dies dadurch erzielt, dass die Bildung von Fibrin verlangsamt oder unterbunden wird. Vorzugsweise enthält das zweite Reagenz zu diesem Zweck als wesentlichen Bestandteil einen Inhibitor für die Gerinnselbildung, beispielsweise die Substanz Pefabloc®FG (Glycyl-1-prolyl-1-arginyl-1-prolinacetat; Pentapharm, Schweiz) oder Harnstoff, ein Harnstoffderivat wie z. B. N-substituierte Harnstoffe, Guanidin, oder ein Guanidinderivat wie z. B. N-substituierte Guanidine, oder Salze dieser Substanzklassen.

Ein weiterer Gegenstand der Erfindung ist ein Reagenz zur Bestimmung eines Gerinnungsparameters aus einer flüssigen Probe. Das Reagenz ist dabei in dem oben beschriebenen erfindungsgemäßen Verfahren einsetzbar.

Das erfindungsgemäße Reagenz enthält einen ersten Reagenzbestandteil, der die Blutgerinnung initiiert (und zu diesem Zweck vorzugsweise einen Initiator/Aktivator des intrinsischen oder extrinsischen Blutgerinnungsweges enthält) und in Kontakt mit der Probe zu einem direkten oder indirekten, nachweisbaren Produkt führt, welches einen Rückschluss auf den Blutgerinnungstatus erlaubt, und einen zweiten Reagenzbestandteil, der die Bildung eines Blutgerinnsels bzw. eine Viskositätszunahme der Probe verlangsamt oder verhindert.

Vorzugsweise erreicht das erfindungsgemäße Reagenz dieses Ziel dadurch, dass der zweite Reagenzbestandteil die Bildung von Fibrin verlangsamt oder unterbindet. Bevorzugt enthält der zweite Reagenzbestandteil zu diesem Zweck als wesentlichen Bestandteil einen Inhibitor für die Gerinnselbildung, beispielsweise die Substanz Pefabloc®FG oder Harnstoff, ein Harnstoffderivat wie z. B. N-substituierte Harnstoffe, Guanidin, oder ein Guanidinderivat wie z. B. N-substituierte Guanidine, oder Salze dieser Substanzklassen.

Bevorzugt enthält der erste oder der zweite Reagenzbestandteil ein Thrombinsubstrat, das zu einem direkt oder indirekt nachweisbaren Produkt führt (s. o.).

Schließlich ist Gegenstand der Erfindung ein Koagulationsmesssystem geeignet zum Ausführen des Verfahrens gemäß einem der Ansprüche 1 bis 10 enthaltend ein Reagenz gemäß einem der Ansprüche 11 bis 16. Bevorzugt enthält das Messsystem dem Fachmann an sich bekannte Komponenten, beispielsweise zum Probentransport, zur Reagenzdosierung und zur Signaldetektion, -verarbeitung und Ergebnisanzeige.

Verfahren und Reagenz der vorliegenden Erfindung sind vorteilhaft nicht nur bei Durchflussmesssystemen, sondern auch bei herkömmlichen, nicht auf die Gerinnselbildung fokussierten Messsystemen, da durch Gerinnselbildung auch andere Nachweisprinzipien nachteilig beeinflusst werden (optisch z. B. Streuung, Adsorption von Reagenz, Beeinträchtigung der Diffusion von elektroaktiven Spezies etc.). Diese Nachteile entfallen natürlich wenn keine Gerinnselbildung stattfindet.

Vorteilhaft am Gegenstand der Erfindung ist, dass außer den Reagenzien keine Wegwerfartikel (Messzellen, Disposables) benötigt werden, da die Koagulationsmessung nicht zu einem Blutpfropfen führt und die Messzelle nach Spülen wieder verwendet werden kann. Ein Auswechseln der Messzelle nach jeder Messung entfällt, so dass eine einfache Möglichkeit zur Automatisierung der Koagulationsmessung besteht. Das erfindungsgemäße Verfahren ist tauglich für die Vollblutmessung; eine aufwändige Probenvorbereitung ist nicht erforderlich. Damit ist das Verfahren prinzipiell auch in bestehende automatisierbare Laborabläufe problemlos integrierbar.

Die Erfindung wird durch die nachfolgenden Beispiele und Figuren näher erläutert.
Figur 1 zeigt schematisch ein Messsystem entsprechend einer ersten beispielhaften Ausführungsform der Erfindung.
Figur 2 zeigt schematisch ein Messsystem entsprechend einer zweiten beispielhaften Ausführungsform der Erfindung.
Figur 3 zeigt schematisch ein Messsystem entsprechend einer dritten beispielhaften Ausführungsform der Erfindung.
Figur 4 zeigt schematisch ein Messsystem entsprechend einer vierten beispielhaften Ausführungsform der Erfindung.
Figur 5 zeigt schematisch ein Messsystem entsprechend einer fünften beispielhaften Ausführungsform der Erfindung.
Figur 6 zeigt die Strom-Zeit-Kurve für eine PT-Messung mit zitriertem Humanplasma (HP) und Preciclot Plus II (P II).
Figur 7 zeigt die Strom-Zeit-Kurve für eine aPPT-Messung mit zitriertem Humanplasma (HP) und Preciclot Plus II (P II).
Figur 8 zeigt die Strom-Zeit-Kurve für ACT-Messungen mit zitriertem Schafblut mit Kaolin-Aktivierung.

Die Ziffern und Abkürzungen in den Figuren haben die folgende Bedeutung:
- ACT_{SB}: Activated Clotting Time für Schafblut
- aPTT_{HP}: aktivierte partielle Thromboplastinzeit für zitriertes Humanplasma
- aPTT_{PII}: aktivierte partielle Thromboplastinzeit für Preciclot Plus II Kontrollplasma
- D: Detektionseinheit
- HP: zitriertes Humanplasma
- I: Strom (in nA)
- M: Mischzone
- P II: Preciclot Plus II Kontrollplasma
- PT_{HP}: Prothrombinzeit für zitriertes Humanplasma
- PT_{PII}: Prothrombinzeit für Preciclot Plus II Kontrollplasma
- R: Reagenz
- R 1: erstes Reagenz
- R 2: zweites Reagenz
- S: Probe
- T: Thermostatisierung
- t: Zeit (in s)
- W: Abfall

### Beispiel 1

### Durchflusskoagulationsmesssystem

Ein erfindungsgemäßes Koagulationsmesssystem, das insbesondere für Durchflussmessungen geeignet ist, enthält typischerweise folgende, mit Schlauch- oder Rohrleitungen verbundene Komponenten:
- Eine Probenflüssigkeit S in einem entsprechenden Behälter (z. B. Kunststoffspritze, Vacutainer, End-to-End-Kapillare etc.);
- Zumindest ein Reagenz R in einem entsprechenden Behälter (z. B. Glas- oder Kunststoffflasche, Reagenzienbeutel etc.);
- Eine Messeinrichtung D (z. B. eine Durchflussmesszelle mit integrierten Elektroden (für elektrochemische Bestimmungen) oder entsprechender Messoptik (Lichtquelle, Lichtdetektor, ggf. Linsensysteme, etc.)), die mit einer entsprechenden Steuer-, Auswerte- und Anzeigeeinheit (typischerweise einem Computer mit Bildschirm) verbunden ist;
- Einen Abfallbehälter W zur Aufnahme der Flüssigkeiten (Probe, Reagenz, Waschflüssigkeiten etc.) nach der Messung;
- Gegebenenfalls eine oder mehrere Mischzonen M (z. B. ausgebildet als Mischkammern oder einfach Schlauch- oder Rohrabschnitten im Flüssigkeitssystem, etc.) zum Vermischen der Probe mit Reagenz, Verdünnungslösungen etc.;
- Gegebenenfalls einer Thermostatisierung (z. B. in Form einer heiz- und/oder kühlbaren Metall-, Keramik- oder Kunststoffplatte), um Probe, Reagenz und Messzelle auf die für die Koagulationsmessung erforderliche Temperatur (typischerweise 37 °C) zu bringen und zu halten;
- Pumpen (z. B. Kolbenpumpen, Peristaltikpumpen etc.) und ggf. Ventile zum Befördern der Flüssigkeiten und Steuern der Flüssigkeitsströme.

Die Komponenten des Messsystems sind für sich genommen dem Fachmann in einer hier nicht erschöpfend darstellbaren Vielfalt bekannt.

Das Fluidiksystem (Behälter, Leitungen, Mischkammer, Messzelle etc.) sollte zumindest in den Bereichen, in denen koagulierbares Material (Probe) transportiert oder gelagert wird, aus gerinnungsneutralen Materialien (z. B. Kunststoff, silanisiertes Glas) gefertigt sein oder zumindest auf den der Probe zugewandten Oberflächen gerinnungsneutral beschichtet sein.

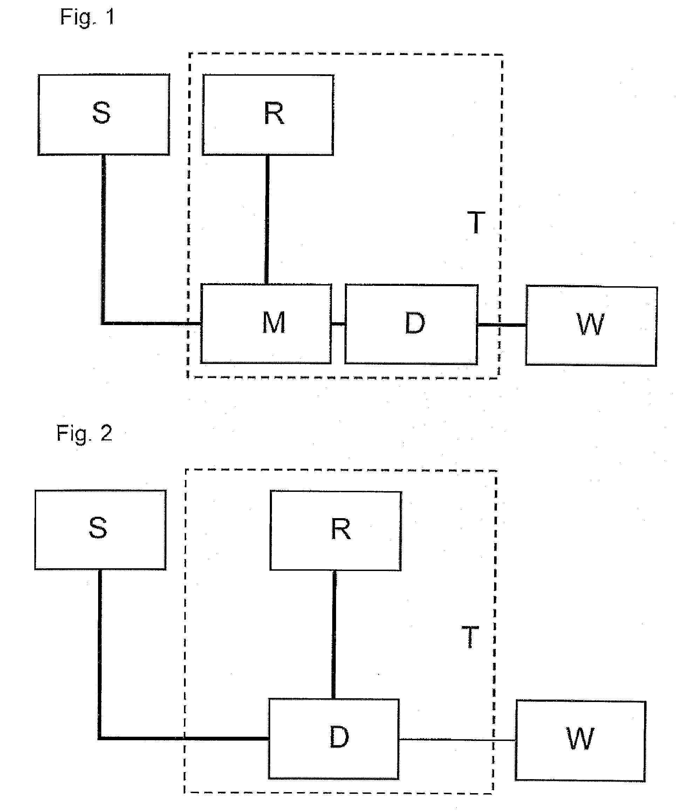

Prinzipiell lässt sich das erfindungsgemäße Verfahren auf vielfältige Weise geräteseitig realisieren. Beispielhaft sind in den Figuren 1 bis 5 fünf prinzipielle Varianten schematisch dargestellt, wobei der Übersichtlichkeit halber gewisse Details und Bestandteile (z. B. Pumpen, Ventile, Behälter für Wasch-/Spülflüssigkeiten, Auswerteeinrichtung etc.) weggelassen wurden. Ohne die Erfindung zu verlassen ist eine Fülle von abgewandelten Varianten denkbar.
a) Fig. 1 zeigt eine erste beispielhafte Ausführungsform des erfindungsgemäßen Messsystems, bei dem ein Reagenz R (enthaltend den ersten und den zweiten Reagenzbestandteil, d. h. zumindest Initiator/Aktivator der Blutgerinnung und Inhibitor der Gerinnselbildung) zur Probe S vor der Messzelle D in einer Mischzone M zugemischt wird. Nach der Messzelle D ist ein Abfallbehälter W vorgesehen. Reagenzbehälter R, Mischzone M und Messzelle D werden dabei durch Thermostat T auf Messtemperatur gehalten. Zur Durchführung der Messung wird das Probe-Reagenz-Gemisch bis zum Erreichen des Endpunktes der Koagulationsmessung in der Messzelle typischerweise nicht bewegt.
b) Fig. 2 zeigt eine zweite beispielhafte Ausführungsform des erfindungsgemäßen Messsystems, bei dem im Unterschied zu dem System gemäß Fig. 1 ein Reagenz R zur Probe S in der Messzelle D zugemischt wird.

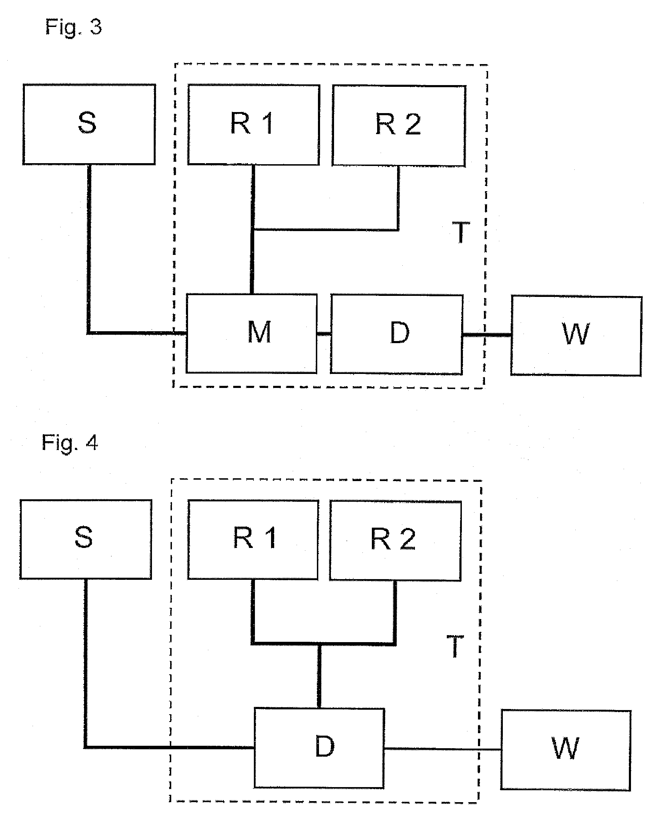

c) Fig. 3 zeigt eine dritte beispielhafte Ausführungsform des erfindungsgemäßen Messsystems, bei dem im Unterschied zu dem System gemäß Fig. 1 zwei Reagenzien R 1 und R 2 (enthaltend einen Initiator/Aktivator der Blutgerinnung bzw. einen Inhibitor der Gerinnselbildung) zur Probe S vor der Messzelle D in einer Mischzone M zugemischt wird.
d) Fig. 4 zeigt eine vierte beispielhafte Ausführungsform des erfindungsgemäßen Messsystems, bei dem im Unterschied zu dem System gemäß Fig. 3 die Reagenzien R 1 und R 2 zur Probe S in der Messzelle D zugemischt werden.

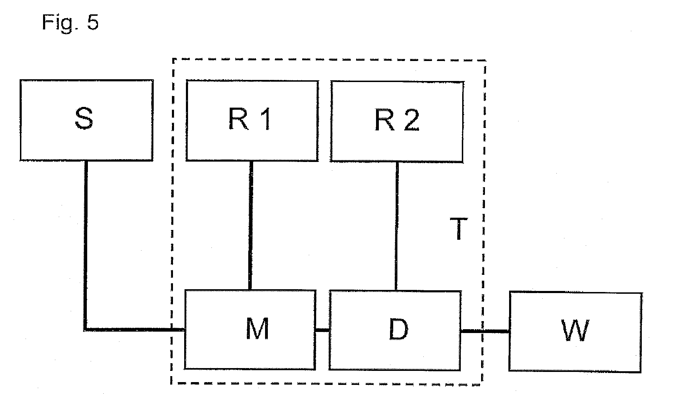

e) Fig. 5 zeigt eine fünfte beispielhafte Ausführungsform des erfindungsgemäßen Messsystems, bei dem im Unterschied zu dem System gemäß Fig. 3 nur ein Reagenz R 1 vor der Messzelle in einer Mischzone M der Probe S zugemischt wird, das zweite Reagenz R 2 jedoch zur Probe S in der Messzelle D zugemischt wird.

Varianten, die ebenfalls erfindungsgemäß umfasst sind, können u. a. sein:
- Die Thermostatisierung T umfasst weniger Bestandteile des Systems als in den Figuren gezeigt (z. B. nur die Messzelle D; nur Messzelle D und Mischzone M; etc.)
- Die Reagenzien R 1 und R 2 werden an unterschiedlichen Stellen in die Messzelle bzw. Mischzone eingespeist.
- Die Reagenzien R1 und R 2 werden in unterschiedliche Mischzonen eingespeist.

### Beispiel 2

### Messung der Prothrombinzeit (PT) mit dem System gemäß Beispiel 1

In das Messsystem gemäß Beispiel 1 (Variante gemäß Figur 3) werden 30 µl Probe (zitriertes Humanplasma bzw. Preciclot Plus II Kontrollplasma (Roche Diagnostics GmbH; für erhöhte Gerinnungszeiten) aufgegeben und 1:1 mit Wasser verdünnt (R 1 gemäß Figur 3 enthält hier nur Wasser!). Zur verdünnten Probe werden 30 µl eines erfindungsgemäßen PT-Reagenzes (STA Neoplastin (Stago; Aktivator, 1 Fläschen Lyophilisat) in 5 ml einer wässrigen 25 millimolaren Kalziumchloridlösung enthaltend 0,3 mmol/l Electrozym TH (Tosyl-glycylprolyl-arginyl-p-aminoanilid, Roche Diagnostics GmbH, Thrombinsubstrat mit elektrochemisch detektierbarer Phenylendiamin-Gruppe) und 0,15 Gew.-% Pefabloc®FG (Pentapharm, Schweiz, Inhibitor)) (R 2) zugemischt (in Mischzone M gemäß Figur 3), und sofort vor einen elektrochemischen Sensor (Siebdruckelektroden auf einem Kunststoffsubstrat (analog zu EP-A 0 603 154; in der Messzelle D gemäß Figur 3) positioniert. Die Zeit zwischen Zumischung des PT-Reagenzes und Start der Messwerterfassung beträgt bei dem System gemäß Beispiel 1 bedingt durch die Schlauchwege ca. 7 Sekunden.

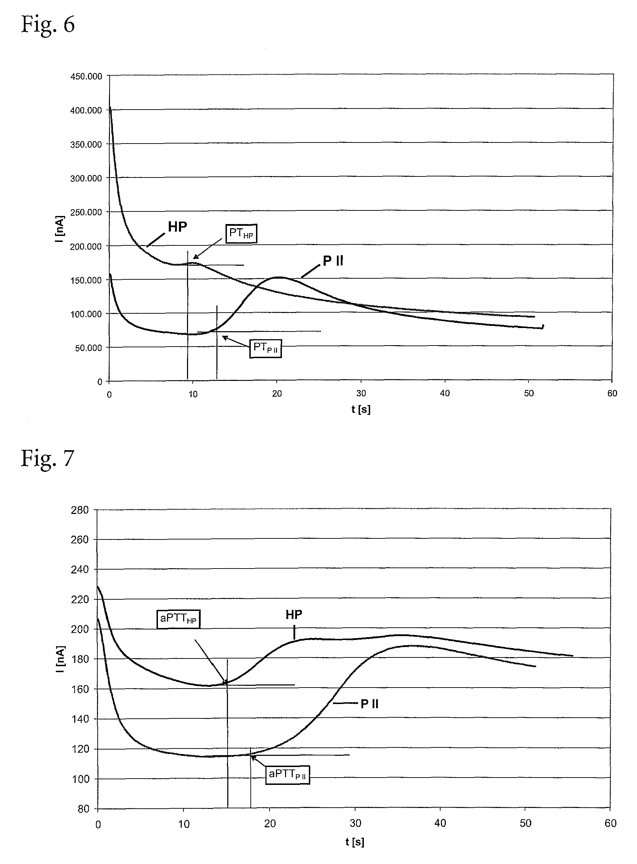

Figur 6 zeigt die PT-Messkurven (Strom I (bei einem Elektrodenpotential von -350 mV gegen eine Silber/Silberchlorid-Referenzelektrode) in nA gegen die Zeit t in s) für die Messung von zitriertem Humanplasma (HP) und Preciclot Plus II (P II), sowie deren grafische Auswertung. Nach dem Abfall des unspezifischen Messstromes zu Beginn der Messung (unmittelbar nach Probenpositionierung vor dem Sensor) kommt es nach 9 (HP) bzw. 12 (P II) Sekunden zum für die thrombinkatalysierte Bildung von Phenylendiamin aus Electrozym TH charakteristischen Stromanstieg. Dieser wird als Endpunkt für die Bestimmung der Gerinnungszeit herangezogen. Die Gerinnungszeit, die sich additiv aus der Zeit zwischen Zumischung des PT-Reagenzes und Start der Messwerterfassung und der gemessenen Zeit bis zum charakteristischen Stromanstieg zusammensetzt, beträgt insgesamt ca. 16 (Humanplasma) bzw. 19 (Preciclot II) Sekunden. Diese Ergebnisse korrelieren gut mit den erwarteten Werten für Normalblut (entsprechend dem zitrierten Humanplasma) und Proben mit erhöhten Gerinnungszeiten (Preciclot Plus II).

Eine Gerinnselbildung in der Probe ist nicht feststellbar. Nach durchgeführter Messung kann die Probe problemlos aus der Messzelle ausgespült werden. Das System steht dann sofort für weitere Messungen zur Verfügung.

Zu Vergleichszwecken durchgeführte Messungen mit dem Reagenz ohne Probe bzw. mit Probe ohne Reagenzzusatz zeigen den für die Thrombinbildung (und damit für die Gerinnungsreaktion) typischen Stromanstieg nicht.

### Beispiel 3

### Messung der aktivierten partiellen Thromboplastinzeit (aPTT) mit dem System gemäß Beispiel 1

Für die Messung der aPTT mit dem Messsystem gemäß Beispiel 1(Figur 3) wird STA Kaolin (Stago, Aktivator) als erstes Reagenz verwendet. Die Herstellung dieses Reagenz erfolgte gemäß Herstellerangaben (Auflösen des Lyophilisates in 10 mL STA Pufferlösung). Als zweites Reagenz dient eine Lösung von 0,3 mmol/l Electrozym TH und 0,15 Gew.-% Pefabloc®FG in 25 mmol/l CaCl2 in Wasser.

In das Messsystem gemäß Beispiel 1 (Figur 3; vgl. auch Beispiel 2) werden 30 µl Probe (zitriertes Humanplasma bzw. Preciclot II Kontrollplasma) aufgegeben und mit 30 µl des ersten Reagenzes (R 1) gemischt. Zum Proben-Reagenz-Gemisch werden nach einer Inkubationszeit von 180 s 30 µl des zweiten Reagenzes (R 2) zugemischt, und die Reaktionsmischung sofort vor den Sensor positioniert. Die Zeit zwischen Zumischung des ersten Reagenzes und Start der Messwerterfassung beträgt bei dem System gemäß Beispiel 1 bedingt durch die Schlauchwege ca. 7 Sekunden.

Figur 7 zeigt die aPTT-Messkurven (Strom I in nA gegen die Zeit t in s) für die Messung von zitriertem Humanplasma (HP) und Preciclot II (P II), sowie deren grafische Auswertung. Nach dem Abfall des unspezifischen Messstromes zu Beginn der Messung (unmittelbar nach Probenpositionierung vor dem Sensor) kommt es nach 15 (HP) bzw. 17,5 (P II) Sekunden zum für die Bildung von Phenylendiamin aus Electrozym TH charakteristischen Stromanstieg. Die Gerinnungszeit die sich additiv aus der Zeit zwischen Zumischung des zweiten Reagenzes und Start der Messwerterfassung und der gemessenen Zeit bis zum charakteristischen Stromanstieg zusammensetzt, beträgt insgesamt ca. 22 (Humanplasma) bzw. 24,5 (Preciclot II) Sekunden. Diese Ergebnisse korrelieren gut mit den erwarteten Werten für Normalblut (entsprechend dem zitrierten Humanplasma) und Proben mit erhöhten Gerinnungszeiten (Preciclot Plus II).

Eine Gerinnselbildung in der Probe ist nicht feststellbar. Nach durchgeführter Messung kann die Probe problemlos aus der Messzelle ausgespült werden. Das System steht dann sofort für weitere Messungen zur Verfügung.

### Beispiel 4

### Messung der Activated Clotting Time (ACT) mit dem System gemäß Beispiel 1

Für die Messung der ACT mit dem Messsystem gemäß Beispiel 1 wird als erstes Reagenz das Kaolin-Reagenz aus den High Range Activated Clotting Time Cartridges (Medtronic) verwendet. Als Probenmaterial dient zitriertes Schafblut. Im Übrigen sind die Zusammensetzung des zweiten Reagenzes und die Durchführung der Messung identisch mit Beispiel 3.

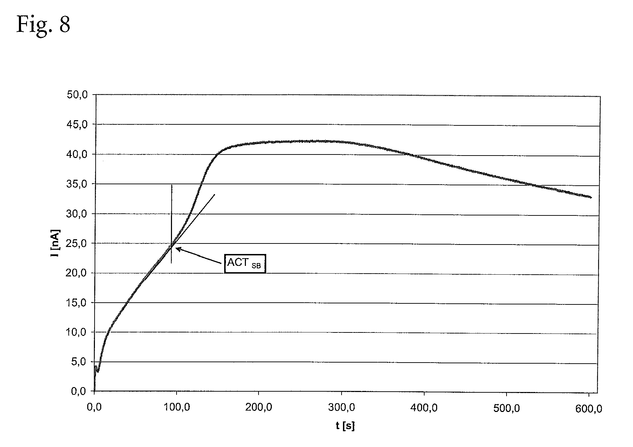

Figur 8 zeigt die ACT-Messkurve für die Messung von Schafblut mit Aktivierung durch Kaolin.

Es ist anhand der Grafik deutlich zu sehen dass es bei Anwesenheit von Kaolin zwischen 100 und 200 Sekunden zu einem deutlichen Anstieg des Stromes kommt (ACT_{SB}, was sehr gut mit der spezifizierten Gerinnungszeit des Probenmaterials von 89 bis 135 Sekunden übereinstimmt.

## Patentansprüche

1. Verfahren zur Bestimmung eines Gerinnungsparameters aus einer flüssigen Probe, wobei
die Probe mit einem ersten Reagenz in Kontakt gebracht wird, welches die Blutgerinnung initiiert und zu einem direkten oder indirekten, nachweisbaren Produkt führt, welches einen Rückschluss auf den Blutgerinnungstatus erlaubt,
**dadurch gekennzeichnet, dass**
der Probe oder dem Probe-Reagenz-Gemisch mindestens ein zweites Reagenz zugesetzt wird, welches die Bildung eines Blutgerinnsels bzw. eine Viskositätszunahme der Probe verlangsamt oder verhindert.

2. Verfahren gemäß Anspruch 1, **dadurch gekennzeichnet, dass** das zweite Reagenz die Bildung von Fibrin verlangsamt oder unterbindet.

3. Verfahren gemäß Anspruch 1 oder 2, **dadurch gekennzeichnet, dass** das zweite Reagenz einen Inhibitor für die Gerinnselbildung enthält.

4. Verfahren gemäß Anspruch 3, **dadurch gekennzeichnet, dass** das zweite Reagenz Glycyl-1-prolyl-1-arginyl-1-prolinacetat (Pefabloc®FG), Harnstoff, ein Harnstoffderivat, Guanidin oder ein Guanidinderivat enthält.

5. Verfahren gemäß Anspruch 1, **dadurch gekennzeichnet, dass** der Gerinnungsparameter Prothrombinzeit (PT), aktivierte Clottingzeit (ACT), aktivierte partielle Thromboplastinzeit (aPTT), Thrombin Generation Test (TGT), Ecarin Clotting Time (ECT), oder Faktor Xa-Test ist.

6. Verfahren gemäß Anspruch 1, **dadurch gekennzeichnet, dass** die Probe antikoaguliertes oder nicht-antikoaguliertes Vollblut oder eine aus Vollblut gewonnene oder von Vollblut abgeleitete Blutfraktion ist.

7. Verfahren gemäß Anspruch 1, **dadurch gekennzeichnet, dass** das erste Reagenz einen Initiator bzw. Aktivator des intrinsischen oder extrinsischen Blutgerinnungssystems enthält.

8. Verfahren gemäß Anspruch 1, **dadurch gekennzeichnet, dass** das erste oder das zweite Reagenz ein Thrombinsubstrat enthält.

9. Verfahren gemäß Anspruch 1, **dadurch gekennzeichnet, dass** das zweite Reagenz der Probe vor, nach oder gleichzeitig mit dem ersten Reagenz zugesetzt wird.

10. Verfahren gemäß Anspruch 1, **dadurch gekennzeichnet, dass** das erste Reagenz das zweite Reagenz enthält.

11. Reagenz zur Bestimmung eines Gerinnungsparameters aus einer flüssigen Probe, enthaltend
einen ersten Reagenzbestandteil, der die Blutgerinnung initiiert und zu einem direkt oder indirekt nachweisbaren Produkt führt, welches einen Rückschluss auf den Blutgerinnungstatus erlaubt,
**dadurch gekennzeichnet, dass**
das Reagenz mindestens einen zweiten Reagenzbestandteil enthält, der die Bildung eines Blutgerinnsels bzw. eine Viskositätszunahme der Probe verlangsamt oder verhindert.

12. Reagenz gemäß Anspruch 11, **dadurch gekennzeichnet, dass** der zweite Reagenzbestandteil die Bildung von Fibrin verlangsamt oder unterbindet.

13. Reagenz gemäß Anspruch 11, **dadurch gekennzeichnet, dass** das zweite Reagenz einen Inhibitor für die Gerinnselbildung enthält.

14. Reagenz gemäß Anspruch 13, **dadurch gekennzeichnet, dass** das zweite Reagenz Glycyl-1-prolyl-1-arginyl-1-prolinacetat (Pefabloc®FG), Harnstoff, ein Harnstoffderivat, Guanidin oder ein Guanidinderivat enthält.

15. Reagenz gemäß Anspruch 11, **dadurch gekennzeichnet, dass** der erste Reagenzbestandteil einen Initiator oder Aktivator des intrinsischen oder extrinsischen Blutgerinnungssystems enthält.

16. Reagenz gemäß Anspruch 14, **dadurch gekennzeichnet, dass** der erste oder der zweite Reagenzbestandteil ein Thrombinsubstrat enthält.

17. Koagulationsmesssystem geeignet zum Ausführen des Verfahrens gemäß einem der Ansprüche 1 bis 10 enthaltend ein Reagenz gemäß einem der Ansprüche 11 bis 15.
